# EUROPEAN PATENT SPECIFICATION

(11) **EP 0 701 836 B1**
(45) Date of publication and mention of the grant of the patent: **31.03.2004**
(21) Application number: 95202504.7
(22) Date of filing: 15.09.1995
(51) Int. Cl.: A61M 25/01

(54) **MR-visible catheter**
MR-sichtbarer Katheter
MR-visible cathéter

(30) Priority: 19.09.1994 NL 9401517
(43) Date of publication of application: 20.03.1996
(73) Proprietor: CORDIS EUROPA N.V., NL-9301 LJ Roden (NL)
(72) Inventor: Weber, Jan, NL-9302 ER Roden (NL); Van Erp, Wilhelmus P. M. M., NL-9351 KS Leek (NL)
(74) Representative: 't Jong, Bastiaan Jacob

(56) References cited:
- WO-A-94/23782
- US-A- 4 003 382
- US-A- 4 827 931
- US-A- 5 154 179

## Description

The invention relates to a catheter comprising a tube-like body extruded from a plastic material with a proximal and a distal end and with at least one lumen. Such catheters are used in medicine for diagnostic and interventional purposes.

With certain applications it is standard practice to make the position of the catheter inside the body of the patient visible by means of X-ray imaging. For this purpose known catheters comprise certain materials, as a result of which they become clearly visible on an X-ray screen.

With other applications, and particularly in the field of neurology, it is desirable to make catheters visible when employing NMR techniques.

The US patent specification 5 154 179 describes a catheter which is visible under such circumstances. Ferromagnetic particles have been incorporated in the material of which the body of the catheter has been made. This is done during extrusion of the body. These particles disturb the magnetic field in the NMR device and result in a deviation in the picture, which is formed, thus indicating the position of the catheter.

The object of the invention is to further improve this known catheter.

According to the invention this is achieved with a catheter as characterised in claim 1. Consequently the catheter becomes clearly visible when using an NMR device.

From the above-mentioned US patent specification the use of paramagnetic material is known as such. In that case the paramagnetic material is however incorporated in a contrast medium, which is introduced into a lumen of the catheter. The catheter according to the invention can effectively have a smaller diameter by incorporating the paramagnetic material, which is used by way of filler, in the plastic material of which the body is made. This is especially important for the neurological applications mentioned before.

Some of the paramagnetic materials suitable for the intended application are harmful to man. In order to be able t use them nevertheless, the measure as set out in claim 1 is employed. A suitable embodiment in that respect is characterised in claim 5.

The visibility of the catheter using MRI-techniques is independent of the direction of the magnetic field when the measures according to claim 2 or claim 3 are applied.

The preferred embodiment as set out in claim 4 allows use of the correct quantity of the paramagnetic material.

In order to also avoid contact of the paramagnetic material at the distal end of the catheter with the body or body fluids of the patient, the measure as set out in claim 6 can be employed in an advantageous manner.

Often catheters are manufactured in such a way that a distal end-section has different material properties, and more in particular greater flexibility, than the basic body. In that case the measure as set out in claim 7 can be employed in an advantageous manner. The end surface of the basic body, which comprises the paramagnetic material, is covered by the end-section, so that there will be no contact with the body or body fluids.

Preferably the measure as set out in claim 8 is employed as well. In that case, one and the same catheter can be used for investigation and/or treatment, employing either an X-ray screen or an NMR screen in order to visualize the catheter.

Materials suitable for application in the catheter according to the invention are characterised in claim 9.

Material which facilitates visibility of the catheter when using x-rays, and which can therefore be used in the catheter according to the invention in an advantageous manner, is characterised in claim 10. The barium- and/or bismuth salts can either be used by way of filler in sections of the plastic material of which the basic body is made or may be arranged as a separate layer or band on the catheter.

The invention will be explained in the following description with reference to the attached drawings.

The figures illustrate preferred embodiments in the corresponding, partly cut away side views. Cross-sectional views are shown to the right of the figures, and these cross-sectional views are taken along the lines indicated with Roman numerals of the same value.

For the sake of simplicity the embodiments shown are all catheters comprising one single lumen, wherein the wall has been drawn comparatively thick. In the drawings dots are used to indicate plastic material containing paramagnetic filler. Hatched areas indicate any other suitable plastic material such as polyurethane or polyethylene.

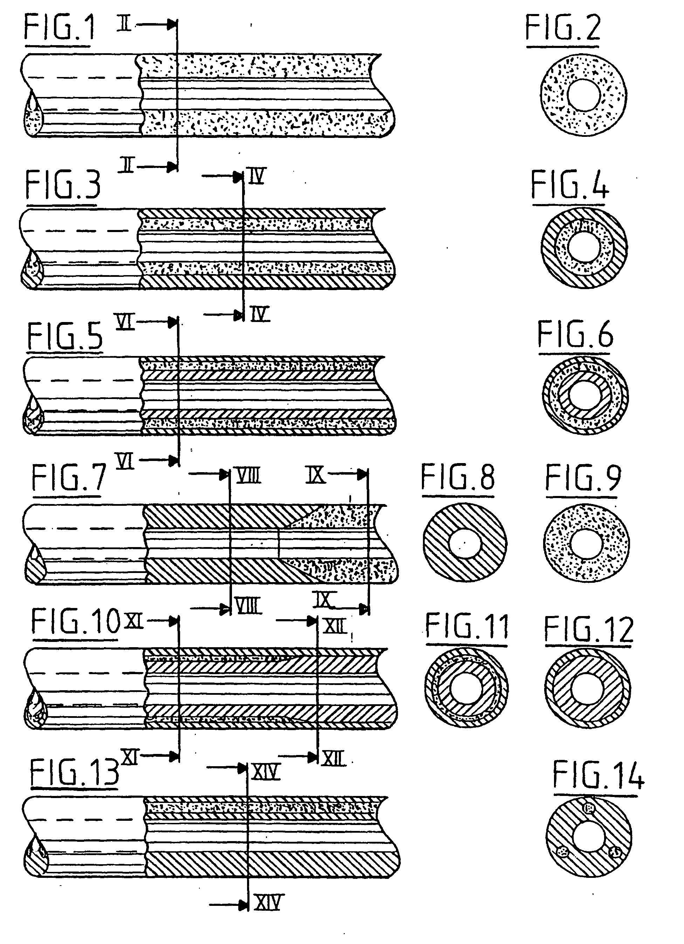

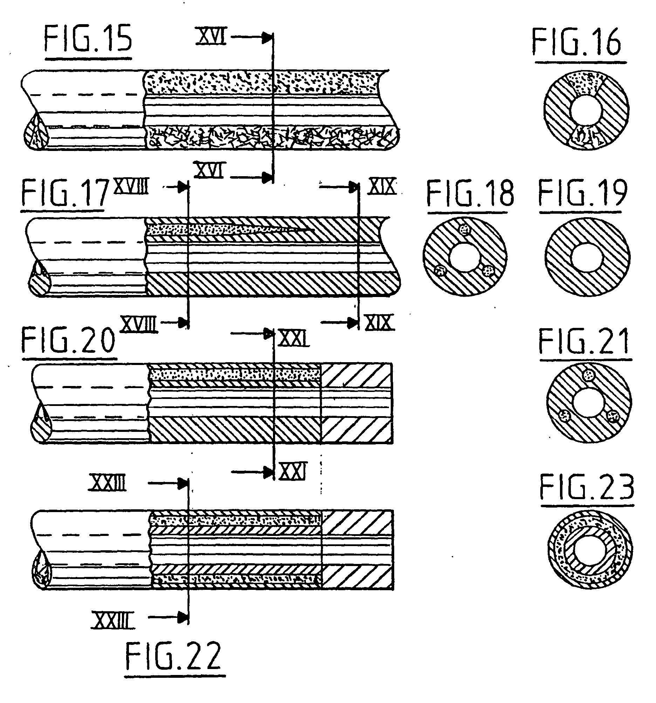

Figure 1 shows the most simple embodiment, wherein the catheter is made up of one type and one layer of plastic material comprising paramagnetic material by way of filler.

Figure 3 shows an embodiment made up of two concentric layers. The inner layer comprises the paramagnetic material, whereas the outer layer is free of paramagnetic material. Consequently there can be no direct contact, from the outside, with the paramagnetic material.

Figure 5 illustrates an embodiment consisting of three lavers. The intermediate layer is made up of the plastic material comprising the paramagnetic material. The inner and the outer layer are both free of paramagnetic material, so that there can be no contact of the body with the paramagnetic material on the outside, and no contact with fluids which are conveyed through the lumen and could end up inside the body of the patient on the inside.

With the embodiment as shown in figure 7, the section comprising a filler of paramagnetic material does not extend over the entire length. The left-hand section is free of paramagnetic material and the right-hand section comprises the plastic material with a filler of paramagnetic material. The two sections may have been manufactured separately and bonded together by glueing for instance.

The choice of an embodiment wherein the paramagnetic material does not extend over the entire length, may be dictated by the intended application. Sometimes it is only necessary to render part of the catheter visible on the NMR screen. If that is the case, it concerns in particular the distal section.

The embodiment of figure 10 also comprises a layer of paramagnetic material only extending over part of the entire length. At the cross-section XI-XI the construction comprises three layers and at the cross-section XII-XII the construction is made up of two layers. The layers may have been formed simultaneously by coextrusion. Ending the intermediate layer with paramagnetic material is achieved by cutting off the supply of the plastic material containing this material by way of filler, during the extrusion process.

The embodiment shown in figure 10 has again the favourable measure that the paramagnetic material cannot come into contact with the surrounding, both on the inside and on the outside of the catheter. For that reason, the paramagnetic material used does not need to be harmless as such.

With the embodiment of figure 13, three strips have been co-extruded in the basic body of the catheter. The material of which these strips are made, is completely surrounded by the basic material of the basic body, so that also in this case there will be no contact with the surrounding.

Figure 15 illustrates an embodiment wherein two strips of different material have been co-extruded in the basic material of the basic body. As mentioned before, the material indicated with dots represents the material comprising paramagnetic material by way of filler. The other layer of different material comprises in this embodiment material which is in particular clearly visible when subjected to X-rays. The material can for instance be a plastic material comprising barium- and/or bismuth salts by way of filler. The catheter thus manufactured is consequently clearly visible both on an NMR screen and on an X-ray screen.

Figure 17 shows an embodiment corresponding to figure 13, wherein the amount of plastic material comprising paramagnetic material reduces from the proximal towards the distal end, close to the distal end, to zero. Also in this embodiment this has been achieved by gradually cutting off the supply of the plastic material comprising the paramagnetic material during the coextrusion process.

With this embodiment of figure 17 there is at the end surface, which corresponds to the cross-section of figure 19, no free paramagnetic material.

With the embodiment of figure 20 the same result has been achieved by connecting a separate end-section of another material, shown on the right, to the end surface of the basic body. Preferably this end-section has been made of a softer plastic material than the basic material, in order to obtain a distal end of the catheter which is as a-traumatic as possible. By ending with the distal end-section, also here no direct contact can be made with the paramagnetic material in the co-extruded strips.

A similar solution is obviously possible with a catheter of the embodiment as shown in figure 5. In figure 22 an end-section of such an embodiment can be seen, which also separates the ring-shaped end surface of the intermediate layer comprising the paramagnetic material from the outside.

Suitable materials for application in the catheter according to the invention are transition metals such as copper, manganese, chromium, nickel gadolinium, dysprosium. Also mixtures, alloys and salts of these materials have suitable properties for application according to the invention. Which quantity of paramagnetic material is to be used in the plastic material by way of filler depends in particular on the properties of the NMR device used for the detection of the catheter.

Also the properties of the paramagnetic material to be used are decisive. With weak paramagnetic materials one will use a stronger concentration of this material than with strong paramagnetic materials. In practice the concentration may vary for instance from 0,001% in the case of strong paramagnetic materials such as dysprosium oxide (DyO₃) to 60% in the case of a weak paramagnetic material such as for instance titanium dioxide. These percentages are percentages by weight.

## Claims

1. A catheter comprising a tubular body extruded from a plastic material with a proximal and a distal end and with at least one lumen, wherein at least part of the plastic material comprises a filler of a paramagnetic material, **characterized in that** the tube-like body comprises at least three coaxial layers and the plastic material comprising the paramagnetic material is an intermediate layer.

2. A catheter as claimed in claim 1, wherein the layer comprising the paramagnetic material forms a helically extending band.

3. A catheter as claimed in claim 1, wherein the layer comprising the paramagnetic material forms a number of spaced apart circular bands.

4. A catheter as claimed in claim 3, wherein the plastic material comprising the paramagnetic material forms at least one strip extending in the longitudinal direction of the tubular body.

5. A catheter as claimed in one of the claims 4, wherein the amount of plastic material comprising the paramagnetic material reduces from the proximal towards the distal end, close to the distal end, to zero.

6. A catheter as claimed in one of the claims 4 and 6, comprising at the distal end an end-section made of a plastic material free of paramagnetic material.

7. A catheter as claimed in one of the previous claims, comprising at least one section made of material visible when subjected to X-rays.

8. A catheter as claimed in claim 7, wherein the material visible when subjected to X-rays is received by way of a filler in a layer of plastic material of the body, which layer also comprises a filler of a paramagnetic material.

9. A catheter as claimed in one of the previous claims, wherein the paramagnetic material has been chosen from the group comprising transition metals such as copper, manganese, chromium, nickel, gadolinium, dysprosium and mixtures, alloys and salts thereof.

10. A catheter as claimed in claim 7, wherein the material visible when subjected to X-rays has been chosen from the group of barium- and bismuth salts.

## Patentansprüche

1. Katheter, aufweisend einen aus einem Plastikmaterial extrudierten rohrförmigen Körper mit einem proximalen und einem distalen Ende und mit zumindest einem Lumen, wobei zumindest ein Teil des Plastikmaterials einen Füllstoff aus einem paramagnetischen Material aufweist, **dadurch gekennzeichnet, dass** der rohrartige Körper zumindest drei koaxiale Schichten aufweist und das paramagnetische Material aufweisende Plastikmaterial eine Zwischenschicht ist.

2. Katheter gemäß Anspruch 1, wobei die das paramagnetische Material aufweisende Schicht ein sich helixförmig erstreckendes Band bildet.

3. Katheter gemäß Anspruch 1, wobei die das paramagnetische Material aufweisende Schicht eine Anzahl von im Abstand voneinander angeordneten kreisförmigen Streifen aufweist.

4. Katheter gemäß Anspruch 3, wobei das paramagnetische Material, welches das Plastikmaterial aufweist, den zumindest einen Streifen bildet, der sich in der Längsrichtung des rohrartigen Körpers erstreckt.

5. Katheter gemäß Anspruch 4, wobei sich die Menge des das paramagnetische Material aufweisenden Plastikmaterials vom proximalen zum distalen Ende hin in der Nähe des distalen Endes bis auf 0 reduziert.

6. Katheter gemäß einem der Ansprüche 4 und 6, aufweisend am distalen Ende einen aus einem Plastikmaterial frei von paramagnetischem Material hergestellten Endabschnitt.

7. Katheter gemäß einem der vorangegangenen Ansprüche, aufweisend zumindest einen Abschnitt, der aus einem Material hergestellt ist, das sichtbar ist, wenn es Röntgenstrahlen ausgesetzt ist.

8. Katheter gemäß Anspruch 7, wobei das Material, das sichtbar ist, wenn es Röntgenstrahlen ausgesetzt ist, in einer Schicht eines Plastikmaterials des Körpers als ein Füllstoff aufgenommen ist, welche Schicht ferner einen Füllstoff aus paramagnetischem Material aufweist.

9. Katheter gemäß einem der vorangegangenen Ansprüche, wobei das paramagnetische Material aus der Gruppe ausgewählt ist, die aus Übergangsmetallen wie Kupfer, Mangan, Chrom, Nickel, Gadolinium, Dysprosium und Gemischen, Legierungen und Salzen davon besteht.

10. Katheter gemäß Anspruch 7, wobei das Material, das sichtbar ist, wenn es Röntgenstrahlen ausgesetzt ist, aus der Gruppe von Barium- und Wismut-Salzen ausgewählt ist.

## Revendications

1. Cathéter comportant un corps tubulaire extrudé à partir d'une matière plastique ayant une extrémité proximale et une extrémité distale et ayant au moins une lumière, au moins une partie de la matière plastique comportant une charge constituée d'un matériau paramagnétique, **caractérisé en ce que** le corps analogue à un tube comporte au moins trois couches coaxiales, et la matière plastique comportant le matériau paramagnétique est une couche intermédiaire.

2. Cathéter selon la revendication 1, dans lequel la couche comportant le matériau paramagnétique forme une bande s'étendant de manière hélicoïdale.

3. Cathéter selon la revendication 1, dans lequel la couche comportant le matériau paramagnétique forme plusieurs bandes circulaires espacées.

4. Cathéter selon la revendication 3, dans lequel la matière plastique comportant le matériau paramagnétique forme au moins un ruban s'étendant dans la direction longitudinale du corps tubulaire.

5. Cathéter selon la revendication 4, dans lequel la quantité de matière plastique comportant le matériau paramagnétique diminue à partie de l'extrémité proximale vers l'extrémité distale, à proximité de l'extrémité distale, jusqu'à zéro.

6. Cathéter selon l'une quelconque des revendications 4 et 6, comportant, au niveau de l'extrémité distale, un tronçon d'extrémité constitué de matière plastique exempte de matériau paramagnétique.

7. Cathéter selon l'une quelconque des revendications précédentes, comportant au moins un tronçon constitué d'un matériau visible lorsqu'il est soumis à des rayons X.

8. Cathéter selon la revendication 7, dans lequel le matériau visible lorsqu'il est soumis à des rayons X est reçu en tant que charge dans une couche de matière plastique du corps, laquelle couche comporte également une charge constituée d'un matériau paramagnétique.

9. Cathéter selon l'une quelconque des revendications précédentes, dans lequel le matériau paramagnétique a été choisi dans le groupe comportant des métaux de transition tels que du cuivre, du manganèse, du chrome, du nickel, du gadolinium, du dysprosium et des mélanges, alliages et sels de ceux-ci.

10. Cathéter selon la revendication 7, dans lequel le matériau visible lorsqu'il est soumis à des rayons X a été choisi dans le groupe comportant des sels de baryum et de bismuth.
